Europäisches Patentamt

European Patent Office

Office européen des brevets

(19)

(11) **EP 0 719 756 B1**

(12) **FASCICULE DE BREVET EUROPEEN**

(45) Date de publication et mention
de la délivrance du brevet:
**31.03.1999 Bulletin 1999/13**

(51) Int Cl.[6]: **C07C 53/08**, C07C 51/215,
B01J 27/199

(21) Numéro de dépôt: **95402918.7**

(22) Date de dépôt: **22.12.1995**

(54) **Procédé de préparation d'acides carboxyliques par oxydation ménagée des alcanes correspondants**

Verfahren zur Herstellung von Carbonsäuren durch Oxydation der entsprechenden Alkane

Process for preparing carboxylic acids by oxidation of corresponding alkanes

(84) Etats contractants désignés:
**BE DE FR GB IT NL**

(30) Priorité: **29.12.1994 FR 9415932**

(43) Date de publication de la demande:
**03.07.1996 Bulletin 1996/27**

(73) Titulaire: **RHODIA CHIMIE**
**92408 Courbevoie Cédex (FR)**

(72) Inventeurs:
• **Aubry, Alain**
**F-94100 Saint Maur des Fosses (FR)**
• **le Govic, Anne-Marie**
**F-75010 Paris (FR)**
• **Gubelmann-Bonneau, Michel**
**F-75016 Paris (FR)**

(74) Mandataire: **Dubruc, Philippe et al**
**RHODIA SERVICES**
**Direction de la Propriété Industrielle**
**25, quai Paul Doumer**
**92408 Courbevoie Cédex (FR)**

(56) Documents cités:
EP-A- 0 627 401     US-A- 4 192 951
US-A- 4 410 752

**Description**

La présente invention a trait à un procédé de préparation d'acides carboxyliques par oxydation ménagée des alcanes correspondants, en présence d'un catalyseur dont la phase active est à base de vanadium, de titane, de molybdène, de phosphore et d'oxygène. Plus particulièrement, l'invention a trait à un procédé de préparation d'acide acétique par oxydation ménagée de l'éthane.

L'obtention d'acides carboxyliques saturés par oxydation ménagée des alcanes correspondants, en présence d'un catalyseur, sont des procédés en plein développement car ils présentent l'avantage d'utiliser des matières premières dont le coût est intéressant.

Parmi les procédés de préparation d'acide acétique notamment connus, on peut citer ceux mettant en jeu, outre l'éthane et l'oxygène, des catalyseurs complexes à base d'au moins un mélange d'oxydes de molybdène, de vanadium, de niobium et/ou d'antimoine. Cependant, la sélectivité en acide acétique par mise en oeuvre de tels catalyseurs, est de l'ordre de 15 à 25 % seulement, alors que les conditions de réactions sont relativement dures. En effet, il est nécessaire d'effectuer l'oxydation dans des conditions combinant une température élevée, c'est-à-dire de l'ordre de 400°C, et une pression d'au moins 20 bar.

Le brevet US 4 192 951 décrit aussi l'emploi de catalyseurs à base de molybdène pour catalyser la réaction d'oxydation du butane en son acide carboxylique insaturé correspondant, l'acide maléique. Il est à noter que de l'acide acétique est de même produit dans cette réaction.

Un autre procédé consiste à faire réagir l'éthane et l'oxygène en présence d'un catalyseur à base au moins d'oxydes de vanadium, de phosphore et de rhénium. Les sélectivités en acide acétique sont améliorées par rapport à celles des catalyseurs précédemment décrits (de l'ordre de 30 %) mais restent cependant inférieures à celles de l'éthylène coproduit. En outre, ce procédé nécessite d'introduire dans le réacteur de fortes quantités d'eau et d'un diluant comme l'hélium ou l'azote.

Il est par ailleurs connu d'utiliser des catalyseurs dont la phase active comprend du vanadium, ce dernier étant au degré d'oxydation (IV), du titane et/ou du phosphore et de l'oxygène. Dans le cas de ces catalyseurs, les performances sont intéressantes en termes de sélectivité en acide acétique. En revanche, les conditions de réactions rendent impossible l'exploitation de ce procédé à l'échelle industrielle. En effet, la conversion de l'éthane est faible et le flux d'alimentation comprend plus de 90 % de gaz diluant, ce qui a pour résultat une productivité très faible.

Un autre type de procédé d'oxydation ménagée, décrit dans la demande de brevet EP 624 401, consiste à effectuer la réaction avec l'éthane, l'oxygène, en présence d'un catalyseur dont la phase active est à base de vanadium (V), de titane et d'oxygène. Cette composition dont l'intérêt n'est pas remis en question, présente toutefois des performances qu'il serait souhaitable d'améliorer, pour une meilleure rentabilité du procédé à l'échelle industrielle.

Un autre type de composition catalytique a été décrit permettant d'obtenir de l'acide acétique par oxydation de l'éthane avec des performances intéressantes.

Cependant, il apparaît que ces dernières soient atteintes en mettant en oeuvre des conditions de réaction relativement dures, puisque la pression est voisine de 30 bar et que la température est proche de 300°C. De plus, la composition catalytique en question ne comprend pas moins de six éléments et le procédé de préparation est particulièrement complexe et difficilement contrôlable en matière d'arrangement des espèces les unes par rapport aux autres dans le catalyseur fini. En effet, la synthèse dudit catalyseur consiste à préparer diverses solutions comprenant, pour la majorité, un seul élément constitutif, puis à évaporer le solvant. Une telle méthode peut conduire à des catalyseurs dont les performances ne sont pas parfaitement reproductibles d'un catalyseur à l'autre.

Ainsi que l'on peut le constater, on ne dispose pas, à l'heure actuelle, de procédés de préparation d'acides carboxyliques par oxydation de l'alcane correspondant, pouvant être développés à l'échelle industrielle et mettant en jeu des catalyseurs dont la synthèse est simple et contrôlée.

Or il a été trouvé de façon totalement inattendue que l'utilisation de catalyseurs à base de vanadium, de titane, de molybdène, de phosphore et d'oxygène, dans la réaction précitée, permettait de pallier les inconvénients mentionnés ci-dessus.

Un avantage supplémentaire du procédé selon l'invention réside dans la température relativement faible d'activation de l'alcane, et plus particulièrement de l'éthane. En effet, les températures classiques nécessaires pour l'activation d'hydrocarbures dans des réactions d'oxydation, telles que l'oxydation du propylène, de l'orthoxylène, du butane sont voisines de 400°C. Sachant que les oléfines sont plus réactives que les composés alkylaromatiques, eux-mêmes étant beaucoup plus réactifs que les alcanes et qu'en outre, la réactivité des hydrocarbures saturés augmente avec le nombre d'atomes de carbone présents dans la molécule, il est tout à fait surprenant de pouvoir activer l'éthane à des températures pouvant être aussi faibles que 150°C-275°C.

Par ailleurs, et ceci représente un autre avantage surprenant, la période d'activation du catalyseur mis en oeuvre selon l'invention est beaucoup plus faible que pour les catalyseurs mis en oeuvre dans les procédés classiques, alors même que la température d'activation est plus faible que dans les procédés classiques.

Ces buts et d'autres sont atteints par la présente invention qui concerne donc un procédé de préparation d'acide carboxylique saturé par réaction en phase gazeuse de l'alcane correspondant, avec une source d'oxygène, en présence d'un catalyseur dont la phase active comprend du vanadium, du titane, du molybdène, du phosphore et de l'oxygène.

Pour plus de commodité, le catalyseur mis en oeuvre dans l'invention va tout d'abord être décrit.

Ainsi que cela a été mentionné auparavant, la phase active du catalyseur utilisé comprend du vanadium, du titane, du molybdène, du phosphore et de l'oxygène.

Selon un mode plus particulier de réalisation de l'invention, la phase active du catalyseur correspond à la formule suivante : $P_aMo_bV_cTi_dO_x$ ; dans laquelle les coefficients stoechiométriques vérifient les relations suivantes : b + c + d = 1,0 ; 0,9 < (a/c) < 3 ; 0,5 < d < 0,99 et 0,01 < (c/b+c) < 0,92.

De préférence, les coefficients stoechiométriques précités vérifient les relations suivantes : b + c + d = 1,0 ; 1,0 < (a/c) < 2,2 ; 0,7 < d < 0,98 et 0,08 < (c/b+c) < 0,6.

La phase active du catalyseur mis en oeuvre dans le procédé de l'invention peut comprendre en outre un dopant.

Le dopant est choisi plus particulièrement parmi les éléments suivants : K, Rb, Cs, Ca, Mg, Zr, Hf, Nb, Ta, Cr, W, Mn, Re, Fe, Ru, Os, Co, Rh, Ir, Ni, Pd, Cu, Ag, Zn, Cd, Tl, Si, Ge, Sn, As, Sb, Bi, Ga et les terres-rares.

De préférence, le dopant est choisi dans la liste suivante : K, Cs, Zr, Nb, Ta, Cr, W, Mn, Re, Fe, Ru, Co, Pd, Ag, Zn, Tl, Sb, Bi, Ga, Sn, La et Ce.

Habituellement, la quantité de dopant dans la phase active est telle que le rapport du dopant au vanadium est compris entre 0,005 et 0,10, et de préférence compris entre 0,009 et 0,06.

Le catalyseur mis en oeuvre dans le procédé de l'invention peut se présenter sous une forme massique, c'est-à-dire comprendre essentiellement la phase active décrite ci-dessus, ou bien encore se présenter sous une forme diluée.

Dans le cas particulier où le catalyseur comprend un diluant (ou aussi support), la phase active peut être soit déposée sur celui-ci, soit l'enrober ou encore y être mélangée.

La nature du diluant n'est pas critique, si ce n'est qu'il doit être inerte vis-à-vis des réactifs dans les conditions réactionnelles choisies.

A titre de matériaux susceptibles d'être utilisés comme support de catalyseur, on peut citer : la silice, l'alumine, la silice-alumine, l'argile frittée, la magnésie, le silicate de magnésium, la terre de diatomée. Ces types de support peuvent être utilisés sous forme poreuse ou non. De préférence, le support utilisé est sous forme non poreuse. Si nécessaire, on peut effectuer un émaillage de ceux-ci afin de le rendre tel.

Les matériaux céramiques, du type de la cordiérite, l'alumine, la mullite, la porcelaine, le nitrure de silicium, les carbures de bore et de silicium, peuvent aussi être utilisés comme diluant.

Le catalyseur mis en oeuvre dans le procédé selon l'invention, dilué ou non, se présente sous forme de particules ou de monolithe.

Dans le cas où le catalyseur est constitué de particules, la granulométrie de celles-ci dépend du mode d'utilisation du catalyseur. Elle peut donc varier dans de larges limites, comme notamment être comprise entre quelques micromètres et une dizaine de millimètres. Plus particulièrement, et à titre indicatif, un catalyseur utilisé en lit fixe, présente une répartition granulométrique généralement comprise entre 0,5 et 6 mm. La granulométrie des particules d'un catalyseur utilisé en lit fluidisé ou mobile, est habituellement comprise entre 5 et 700 microns, et de préférence comprise entre 5 et 200 microns pour 80 % des particules.

D'une façon classique, la quantité de diluant entrant dans la composition du catalyseur varie dans de larges limites, dépendant, la plupart du temps, du mode de mise en forme du catalyseur.

Ainsi, les catalyseurs obtenus par enrobage ou dépôt de la phase active sur le support présentent une quantité de phase active variant habituellement entre 0,1 et 30 % et de préférence entre 2 et 20 % en poids total de catalyseur fini (phase active et support).

Dans les cas où le catalyseur comprend d'un support dispersé dans la phase active, la quantité de phase active est comprise en général entre 1 et 90 % en poids total de catalyseur fini.

Selon un mode de réalisation particulier de l'invention, la réaction est réalisée en présence d'un catalyseur de type enrobé.

La phase active du catalyseur mis en oeuvre dans la présente invention peut être obtenue par toute méthode connue de l'homme du métier.

On peut envisager par exemple de fabriquer ladite phase active par mélange des oxydes des éléments constitutifs de celle-ci, suivi d'une étape de calcination, éventuellement suivie et/ou précédée par un broyage dudit mélange (technique de chamottage).

Une autre méthode convenable pour la préparation de la phase active consiste à effectuer le séchage d'une solution des éléments constitutifs de la phase active, suivie d'une calcination. On peut de même envisager d'effectuer cette opération de séchage sur une suspension desdits éléments constitutifs, cette suspension provenant de l'emploi d'au moins l'un de ces éléments sous une forme de solide ou encore provenant d'une étape intermédiaire de précipitation, ou d'hydrolyse contrôlée d'un ou plusieurs alcoxydes par exemple.

Par éléments constitutifs, on désigne non seulement le vanadium, le titane, le phosphore et le molybdène mais aussi le ou les dopants ajoutés à la composition de la phase active.

Habituellement, les éléments constitutifs sont utilisés sous la forme d'une solution ou d'une suspension.

D'une façon avantageuse, le milieu dispersant (ou solubilisant) est l'eau, bien que tout autre type de dispersant (ou solvant) soit envisageable. A ce titre, on peut citer notamment les alcools, comme le méthanol, l'éthanol, l'isopropanol, le tertiobutanol.

Les éléments constitutifs de la phase active entrant dans la composition du mélange précité, sont utilisés généralement sous forme de sels d'acides ou de bases inorganiques ou organiques, ou encore sous forme de composés comme les oxydes ou leurs dérivés.

Tous les acides ou dérivés d'oxydes indiqués conviennent à la préparation du mélange dans la mesure où ceux-ci peuvent se décomposer en oxyde du ou des éléments correspondants.

A titre d'exemples de sels inorganiques convenant à la préparation du mélange précité, on peut mentionner entre autres les nitrates, les sulfates, les halogénures comprenant un ou plusieurs halogènes, les sels d'ammonium.

A titre d'exemples de sels d'acides ou d'esters organiques, on peut mentionner le formiate, l'oxalate, le tartrate, l'acétate, l'acétylacétonate, l'éthylhexanoate.

Ainsi qu'il a été indiqué auparavant, il est aussi envisageable d'utiliser des oxydes ou leurs dérivés ; ces composés pouvant être utilisés sous forme de particules, ou sous forme solubilisée, notamment par ajout d'un acide ou d'une base au dit mélange.

Par dérivés d'oxydes, on entend les composés du type des oxyhalogénures, des alkoxydes, les aryloxydes, les glycoxydes notamment.

Il est à noter que tous ces types de composés peuvent être utilisés seuls ou en mélange.

A titre d'exemples de composés comprenant du vanadium, convenant à la mise en oeuvre de ce mode de préparation, on peut citer, sans intention de se limiter, le sulfate de vanadyle, le métavanadate d'ammonium, les oxyhalogénures de vanadium tels que, notamment, $VOCl_3$, $(VO_2)Cl$, $VOCl$, $VOBr$, $VOBr_2$, $VOF_3$, $VOF_2$, $VF_4$, $VBr_2$, $VI_2$, l'acétylacétonate de vanadyle, l'oxalate de vanadyle, l'acide métavanadique, l'hexacarbonyle de vanadium, le tri-isopropoxyde d'oxyde de vanadium, les oxydes de vanadium comme par exemple, $V_2O_5$, $V_7O_{13}$, $VO$, $VO_2$, $V_2O_3$, $V_3O_7$, ou leurs mélanges.

En tant que composé comprenant du titane, on peut citer les composés du type $TiX_4$ avec X représentant un halogène et plus particulièrement le chlore, les composés du type $Ti(OR)_4$ avec R représentant un groupement alkyle et plus particulièrement les radicaux éthyle, isopropyle ou encore sec-butyle.

Convient aussi à la mise en oeuvre de l'invention l'oxyde de titane sous ses diverses formes allotropiques, c'est-à-dire sous forme anatase, rutile, brookite ou bronze (symbolisé (B)), ou leurs mélanges. De préférence, la forme allotropique de l'oxyde de titane est choisie parmi les formes anatase, rutile, ou leurs mélanges.

SI l'on met en oeuvre la synthèse de la phase active avec un oxyde de titane, on utilise plus spécialement un oxyde de titane dont la surface spécifique, mesurée selon la méthode B.E.T., est comprise entre 1 et 150 $m^2$/g. Plus particulièrement la surface spécifique est comprise entre 10 et 120 $m^2$/g.

Parmi les composés susceptibles de convenir à l'invention à base de molybdène, on peut citer notamment le dimolybdate d'ammonium, l'heptamolybdate d'ammonium, le paramolybdate d'ammonium, l'acétylacétonate de dioxomolybdène.

En ce qui concerne les composés à base de phosphore, on peut citer à titre d'exemple les acides phosphoriques, comme l'acide orthophosphorique, l'acide pyrophosphorique, l'acide métaphosphorique, l'acide polyphosphorique, les phosphates d'alkyles comme le méthyle, l'éthyle, le butyle, et les phosphates d'ammonium.

Les composés apportant le ou les éléments employés en tant que dopants peuvent notamment être choisis parmi les chlorures de potassium, l'acétate de potassium, les chlorures de rubidium, le chlorure ou l'oxychlorure de niobium, l'oxalate de niobium, le sulfate de césium, l'acétate de césium, le sulfate de fer, l'acétate de fer, les chlorures ou chlorates de chrome, les nitrates de chrome, l'acétate de chrome, l'acétylacétonate de chrome, le métatungstate et le paratungstate d'ammonium, les oxydes et alkyloxydes, comme l'éthoxyde, de zirconium, l'oxyde d'argent.

Bien entendu, ces listes ne peuvent être considérées comme exhaustive.

Selon un mode de réalisation particulier de l'invention, on emploie le titane sous la forme d'un oxyde. Dans ce cas, on effectue au moins une imprégnation de l'oxyde avec au moins une solution comprenant les éléments constitutifs de la phase active. On peut ainsi effectuer une seule opération d'imprégnation ou une succession d'étapes d'imprégnation. Les opérations d'imprégnation sont réalisées de manière connue en elles-mêmes. Cependant, on met en oeuvre plus particulièrement au moins une étape d'imprégnation à sec. Cela signifie que le volume total de la solution d'imprégnation mis en oeuvre doit être égal au volume poreux total du solide à imprégner.

Tous les éléments constitutifs de la phase catalytique peuvent être mis en contact simultanément ou successivement avec le support.

Selon une première variante, on met en contact le support avec les éléments constitutifs autres que le phosphore ; ces éléments étant apportés dans un ordre quelconque, simultanément ou successivement. Puis on effectue une l'imprégnation de l'ensemble avec une solution apportant le phosphore.

Une seconde variante consiste à procéder à l'inverse. Ainsi on effectue en premier lieu une ou plusieurs opérations d'imprégnation de l'oxyde de titane avec une solution comprenant le phosphore puis au moins une étape d'imprégnation avec les éléments restants, ces derniers pouvant être apportés avec des solutions distinctes simultanément ou successivement, ou encore avec la même solution.

Bien entendu, il est possible d'introduire le composé du phosphore entre chacune des imprégnations avec les composés du molybdène et du vanadium.

On procède ensuite à une ou plusieurs étapes de séchage.

Selon la méthode employée pour la mise en contact des éléments constitutifs, on réalise le séchage en une ou deux étapes essentiellement.

Ainsi, dans le cas où le procédé de préparation de la phase catalytique met en oeuvre des suspensions ou des solutions, le séchage est effectué en deux temps. Le premier consistant à évaporer le solvant ou dispersant du mélange, jusqu'à siccité, et le second à sécher la pâte ainsi obtenue. Généralement, la première étape est réalisée à une température variant de 20 à 100°C pendant la durée nécessaire pour obtenir une pâte non coulante. L'évaporation est habituellement effectuée sous agitation.

La pâte résultante est ensuite séchée, dans un second temps, sous une atmosphère de préférence non réductrice, comme l'oxygène ou l'air par exemple, pendant une durée moyenne de 15 heures.

La température de séchage est habituellement d'environ 120°C.

On ne sortirait pas du cadre de l'invention en procédant à un séchage par atomisation, méthode bien connue de l'homme du métier. Ainsi, sans intention de se limiter, les atomiseurs de type BUCHI ou encore les atomiseurs de type "flash" comme revendiqués dans les demandes de brevets français publiées sous les numéros suivants : 2 257 326, 2 419 754, 2 431 321, conviennent à ce mode de réalisation. La température d'atomisation est généralement de l'ordre de 150 à 300°C. L'atmosphère sous laquelle est effectuée l'atomisation, là encore, est de préférence non réductrice. De façon avantageuse, on effectue l'atomisation sous air, bien que l'oxygène soit envisageable pour une telle étape.

Dans le cas où les éléments constitutifs de la phase active ont été mis en contact par imprégnation à sec, on effectue le séchage en une seule étape correspondant à la seconde étape précitée de la variante précédente. Il est à noter que de préférence, on effectue une étape de séchage intermédiaire après chaque opération d'imprégnation à sec.

Le produit séché obtenu, quelle que soit la méthode employée, est ensuite soumis à une étape de calcination.

Celle-ci est réalisée, de façon classique, sous une atmosphère non réductrice. Avantageusement on utilise l'air, mais l'oxygène pourrait tout aussi bien être employé.

La température de calcination est habituellement comprise entre 200 et 1200°C.

Généralement, la durée de l'opération varie entre 1 et 24 heures.

Préalablement à l'étape de calcination, le produit séché peut subir une étape de broyage. Il est de plus précisé que le produit calciné peut éventuellement subir aussi un tel traitement.

Ainsi que cela a été mentionné auparavant, le catalyseur mis en oeuvre dans le procédé selon la présente invention peut comprendre ou non un diluant (ou support) enrobé ou mélangé à la phase active.

D'une façon classique, la quantité de diluant entrant dans la composition du catalyseur varie dans de larges limites, dépendant, la plupart du temps, du mode de mise en forme du catalyseur.

Le catalyseur selon l'invention peut être obtenu selon toute méthode classique connue de l'homme du métier.

Ainsi, les catalyseurs massiques, comprenant essentiellement la phase active telle que définie plus haut, peuvent être mis en forme par extrusion, par moulage, par broyage, concassage ou tout autre moyen, de la phase active ou de son précurseur, de façon à donner un monolithe ou encore des particules de granulométrie convenable.

Ici et pour tout le reste de la description, on entend par précurseur de la phase active, le mélange des éléments constitutifs de cette phase dans tous les états antérieurs à l'étape de calcination décrite auparavant.

Dans le cas de catalyseurs comprenant un diluant, les moyens décrits précédemment sont utilisables. Ainsi, on peut mélanger la phase active avec la proportion requise de diluant, et par exemple, extruder ou mouler le mélange résultant.

Cependant d'autres méthodes sont envisageables.

Ainsi, selon un premier mode de réalisation, on met en contact le diluant, de préférence sous forme de particules rugueuses, et la phase active ou son précurseur, dans un mélangeur à fort cisaillement (appareils type LODIGE) ou dans un appareil de granulation (drageoirs, sous forme de tambour ou assiette).

L'opération est effectuée en général à une température variant entre 20 et 150°C pendant la durée nécessaire à l'enrobage du support par la quantité désirée de phase active, plus particulièrement sous air, pendant au moins 30 minutes.

Les particules ainsi obtenues sont habituellement calcinées à une température comprise entre 300 et 600°C, de préférence entre 450 et 500°C.

La durée de la calcination est généralement d'au moins 3 heures.

Un second mode possible de fabrication du catalyseur consiste à mettre en application la technique d'imprégnation.

Selon cette technique, on imprègne le support avec une suspension de la phase active ou avec une suspension ou solution de son précurseur.

L'étape d'imprégnation est suivie d'une étape de séchage, habituellement effectuée à une température comprise entre 100 et 200°C, sous air, pendant au moins 30 minutes.

On peut ensuite renouveler le cycle imprégnation - séchage et terminer celui-ci par une calcination sous air.

La température de calcination est comprise entre 400 et 600°C pendant une dizaine d'heures.

Une variante possible consiste à effectuer une calcination entre le ou les cycles imprégnation-séchage.

Selon un troisième mode de préparation du catalyseur, on ajoute au mélange d'au moins l'un des éléments constitutifs de la phase active, le support, préférentiellement sous forme de particules. Le mélange ainsi obtenu est ensuite traité conformément aux divers modes de réalisation du procédé de préparation de la phase catalytique, tels que décrits auparavant.

Bien entendu, tous ces modes de préparation ne sont donnés qu'à titre indicatif et ne peuvent en aucun cas constituer une liste exhaustive.

La présente invention concerne par ailleurs un procédé de préparation d'acides carboxyliques par réaction en phase gazeuse, des alcanes correspondants avec une source d'oxygène, en présence d'un catalyseur tel que défini précédemment.

Plus particulièrement, la présente invention est adaptée à l'obtention d'acides carboxyliques saturés présentant 1 à 4 atomes de carbone à partir de l'alcane correspondant. Par alcane, on entend les hydrocarbures saturés présentant 1 à 4 atomes de carbone, ces derniers pouvant ou non être substitués par un ou plusieurs atomes d'halogène.

D'une façon avantageuse, le procédé selon l'invention permet d'obtenir de l'acide acétique par réaction de l'éthane.

Il n'y a pas de conditions particulières concernant la qualité de l'alcane mis en oeuvre. Cependant, pour des questions évidentes de séparation de l'acide formé, on préfère utiliser un alcane présentant une pureté d'au moins 90 %.

Ce dernier peut être utilisé indifféremment seul ou dilué dans un gaz diluant, inerte dans les conditions de la réaction. Les gaz rares, comme notamment l'hélium ou l'argon, ou encore l'azote sont des gaz diluants convenant à la mise en oeuvre du procédé selon l'invention.

La réaction d'oxydation ménagée de l'alcane est mise en oeuvre en présence d'une source d'oxygène. Celle-ci peut être à base d'oxygène pur ou dilué dans un gaz inerte. Ainsi on peut effectuer la réaction d'oxydation en utilisant de l'air comme source d'oxygène.

Selon un mode de réalisation particulier de la présente invention, le rapport molaire entre l'alcane et l'oxygène est inférieur à 20. Plus particulièrement, ce rapport est compris entre 0,01 et 0,2 ou entre 0,6 et 15.

Selon un mode préféré de l'invention, ledit rapport est compris entre 0,6 et 15.

Une variante du procédé consiste à utiliser un mélange gazeux comprenant, outre les autres constituants, de l'eau.

La composition du mélange gazeux, soit l'alcane, la source d'oxygène, le cas échéant le gaz diluant et l'eau, peut varier dans de larges limites.

Sauf mention contraire, tous les pourcentages indiqués par la suite, sont exprimés en nombre de moles total du mélange gazeux.

D'une façon générale, la teneur en éthane dans le mélange gazeux, est comprise entre 0,1 et 99,9%.

Selon un mode particulier de réalisation de l'invention, la composition du mélange gazeux est telle qu'elle se trouve en dehors de la zone d'explosivité dudit mélange.

Ainsi, afin d'avoir un mélange gazeux dont la composition se trouve commodément en dehors du domaine d'explosivité, ladite teneur en alcane est plus particulièrement comprise entre 0,1 et 3 % ou entre 10 et 99 %.

Préférentiellement, la teneur en alcane dans le mélange gazeux précité est comprise entre 10 et 99 %.

La teneur en oxygène dans le mélange gazeux mis en oeuvre varie de même dans une large gamme de concentration. Elle est en effet comprise entre 0,1 et 99,9 %.

Selon un mode de réalisation plus particulier, la teneur en oxygène dans le mélange gazeux varie entre 1 et 90 % ou entre 97 et 99,9%.

De préférence, l'oxygène contenu dans ledit mélange est comprise entre 1 et 90%.

La teneur en eau dans le mélange gazeux mis en oeuvre est comprise entre 0 et 70 %.

Selon un mode de réalisation particulier, la teneur en eau dans le mélange précité est de 0 à 20%.

La teneur en gaz diluant dans le mélange, varie habituellement entre 0 et 70%.

Plus particulièrement le mélange comprend entre 0 et 20 % de gaz diluant.

Le mélange gazeux est donc mis au contact du catalyseur selon l'invention.

Le dispositif dans lequel le procédé selon l'invention est mis en oeuvre fait partie des dispositifs classiques pour les réactions catalytiques en phase gaz ; ces derniers pouvant être utilisés en continu ou en discontinu.

Ainsi, on peut effectuer la réaction en présence d'un catalyseur en lit fixe, fluidisé ou encore transporté.

La température de réaction est en général située entre 100 et 350°C, de préférence entre 150 et 330°C.

La pression totale du mélange gazeux réactionnel varie en général entre 0,1 et 30 bar absolus. Plus particulièrement, la pression varie entre 0,1 et 20 bar absolus. De préférence, et d'une façon avantageuse, la pression mise en jeu dans la réaction est comprise entre 1,1 et 10 bar absolus.

Le débit gazeux est fixé de telle sorte que le temps de contact, calculé dans les conditions normales de température et pression, soit compris entre 0,1 et 30 secondes. De préférence, le temps de contact est compris entre 0,5 et 20 secondes. Il est rappelé que le temps de contact correspond au rapport entre le volume du réacteur et le débit total des gaz.

L'acide formé est séparé des sous produits ou des réactifs, classiquement par refroidissement puis condensation d'un mélange acide/eau. Les composés restés sous forme gazeuse, plus particulièrement l'alcane, peuvent être recyclés au réacteur, après avoir subi éventuellement une étape de séparation.

Des exemples concrets mais non limitatifs de l'invention vont maintenant être présentés.

## EXEMPLE 1 COMPARATIF

### Préparation du catalyseur 1

90 g de $TiO_2$ (soit 1,125 mol), présentant une surface BET égale à 86 $m^2/g$ (commercialisé par Rhône-Poulenc) sont introduits dans un drageoir et imprégnés à sec par 100 $cm^3$ d'une solution d'oxalate de vanadyle.

La solution utilisée comprend 15,23 g d'acide oxalique dihydrate (soit 120,8 mmol) et 6,77 g de $V_2O_5$ commercial de JANSSEN (soit 37,2 mmol) dans l'eau.

Le produit ainsi obtenu est calciné à l'air à 500°C, pendant 3 heures.

La composition du produit calciné présente 7% en poids de $V_2O_5$ et un rapport V / Ti de 0,066.

## EXEMPLE 2 COMPARATIF

### Préparation du catalyseur 2

On dissout 3,15 g d'acide tartrique à chaud sous agitation dans 17 ml d'eau permutée. Après dissolution, on ajoute à la solution 1,40 g de $V_2O_5$ en poudre. Ce composé est dissous à chaud et sous agitation.

Une fois obtenue une solution, on laisse cette dernière revenir à température ambiante puis on ajoute à celle-ci 2,03 g d'acide orthophosphorique à 85 %.

45 g de dioxyde de titane sous forme anatase (surface spécifique : 45 $m^2/g$, volume poreux total : 0,38 $cm^3/g$) sont alors imprégnés par cette solution dans un drageoir tournant.

On sèche la poudre obtenue à l'air pendant 6 heures à 150 °C puis on effectue une calcination pendant 3 heures sous air à 400°C.

On obtient ainsi un solide présentant un rapport atomique P/V/Ti=0,031/0,027/1.

## EXEMPLE 3

### Préparation du catalyseur 3

On dissout au reflux, sous agitation, dans un volume de 70 $cm^3$ d'eau désionisée, un mélange de 16,8 g d'heptamolybdate d'ammonium de formule $(NH_4)_6Mo_7O_{24}$ (soit 0,095 mole de Mo) et 1,11 g de métavanadate d'ammonium de formule $NH_4VO_3$ (soit 0,0095 mole de V).

La solution jaune obtenue est refroidie et son volume est ajusté à 80 $cm^3$.

Cette solution est utilisée pour imprégner à sec 50 g de $TiO_2$ anatase présentant une surface spécifique de 80 $m^2/g$ et un volume poreux de 0,8 $cm^3/g$( soit 0,625 mole de Ti).

L'imprégnation est réalisée en deux fois, avec mise en jeu de 40 $cm^3$ de solution à chaque opération et séchage intermédiaire à 110°C.

On effectue ensuite une imprégnation à sec du solide résultant avec 40 $cm^3$ d'une solution aqueuse d'acide phosphorique contenant $9,52.10^{-3}$ mole de $H_3PO_4$.

Après séchage à 110°c sous air, on effectue une calcination du solide résultant à 400°C pendant 2 heures sous air.

On obtient ainsi un solide présentant un rapport atomique

P/Mo/V/Ti=0,015/0,153/0,014/1.

## EXEMPLE 4

Mise en oeuvre des catalyseurs

Cet exemple illustre l'application des catalyseurs précédemment préparés, dans un procédé de préparation d'acide acétique à partir d'éthane.

1 à 3 grammes de catalyseur en poudre broyé (granulométrie inférieure à 200 µm) sont introduits dans un réacteur continu en Hastelloy C22, à lit fixe. Le volume du lit catalytique résultant est de 5 cm$^3$, cette valeur étant obtenue au besoin en diluant le catalyseur avec du carbure de silicium de même granulométrie. Les volumes morts en amont et en aval du lit catalytique sont également garnis par du carbure de silicium.

Le réacteur est équipé d'un chauffage par bain de sable fluidisé et de deux chromatographes en ligne, l'un fonctionnant avec un détecteur à ionisation de flamme et l'autre d'un détecteur thermoconductimétrique.

Les gaz d'alimentation sont contrôlés par des débitmètres massiques.

L'eau est introduite sous forme liquide (pompe doseuse GILSON) dans un vaporiseur intégré au circuit gazeux.

Le flux d'alimentation est constitué de :

$C_2H_6/O_2/N_2/H_2O$=62/17/10/12 % mol.

Le débit d'éthane, rapporté à la masse de catalyseur employé est de 2,0 g/l/h dans les conditions normales de température et de pression.

La température est de 275°C.

Le tableau suivant rassemble les résultats obtenus.

Dans celui-ci, les performances sont calculées de la manière suivante :

- conversion de l'éthane (% mol)

$$\text{conv. éthane} = \frac{\text{(nbre mole éthane entrée - nbre mole éthane sortie)}}{\text{(nbre mole éthane entrée)}} \times 100$$

- sélectivité d'un produit X (acide acétique, éthylène, produits de comb.) (% mol)

$$\text{sél. X} = \frac{\text{nbre mole éthane converti en X}}{\text{(nbre mole éthane entrée - nbre mole éthane sortie)}} \times 100$$

| CATALYSEUR | p (bar abs.) | CONVERSION ETHANE (%) | SELECTIVITE (%) | | |
|---|---|---|---|---|---|
| | | | acide acétique | éthylène | produits de combustion |
| 1 comparatif | 1 | 0,3 | 5 | 40 | 55 |
| | 7 | 2,2 | 15 | 20 | 65 |
| 2 comparatif | 1 | 0,7 | 17 | 33 | 49 |
| | 6 | 3,6 | 20 | 18 | 61 |
| 3 invention | 1 | 1,0 | 28 | 33 | 39 |
| | 6.3 | 6.5 | 38 | 11 | 51 |

## Revendications

1.   Procédé de préparation d'un acide carboxylique saturé par réaction en phase gazeuse de l'alcane correspondant avec une source d'oxygène, caractérisé en ce que l'on effectue la réaction en présence d'un catalyseur dont la phase active est à base de vanadium, de titane, de molybdène, de phosphore et d'oxygène.

**2.** Procédé selon la revendication précédente, caractérisé en ce que l'on effectue la réaction en présence d'un catalyseur dont la phase active correspond à la formule suivante : $P_aMO_bV_cTi_dO_x$ ; dans laquelle les coefficients stoechiométriques vérifient les relations suivantes :

$$b + c + d = 1,0;$$

$$0,9 < (arc) < 3 ;$$

$$0,5 < d < 0,99$$

et

$$0,01 < (c/b+c) < 0,92.$$

**3.** Procédé selon la revendication précédente, caractérisé en ce que les coefficients stoechiométriques vérifient les équations suivantes :

$$b + c + d = 1,0;$$

$$1,0 < (a/c) < 2,2 ;$$

$$0,5 < d < 0,98$$

et

$$0,08 < (c/b+c) < 0,6.$$

**4.** Procédé selon l'une quelconque des revendications précédentes, caractérisé en ce que l'on utilise un catalyseur sous forme massique ou diluée.

**5.** Procédé selon la revendication précédente, caractérisé en ce que l'on utilise un catalyseur enrobé comprenant 0,1 à 30 % en poids de phase active, par rapport au poids total de catalyseur fini, et de préférence, de 2 à 20 % en poids.

**6.** Procédé selon la revendication 4, caractérisé en ce que l'on utilise un catalyseur comprenant un support dispersé dans la phase active, comprenant 1 à 90 % de phase active par rapport au poids total du catalyseur fini.

**7.** Procédé selon l'une quelconque des revendications précédentes, caractérisé en ce que l'on utilise un catalyseur dont la phase active comprend en outre au moins un dopant choisi parmi les éléments suivants : K, Rb, Cs, Ca, Mg, Zr, Hf, Nb, Ta, Cr, W, Mn, Re, Fe, Ru, Os, Co, Rh, Ir, Ni, Pd, Cu, Ag, Zn, Cd, Tl, Si, Ge, Sn, As, Sb, Bi, Ga et les terres-rares.

**8.** Procédé selon la revendication précédente, caractérisé en ce que la teneur en dopant est telle que le rapport du dopant au vanadium est compris entre 0,005 et 0,10, et de préférence compris entre 0,009 et 0,06.

**9.** Procédé selon l'une quelconque des revendications précédentes, caractérisé en ce que l'on effectue la réaction avec un mélange gazeux comprenant 0,1 à 99,9 % en mole d'alcane, et plus particulièrement ledit mélange comprend entre 0,1 et 3 % ou entre 10 et 99 % en mole d'alcane.

**10.** Procédé selon l'une quelconque de revendications précédentes, caractérisé en ce que l'on utilise comme source d'oxygène de l'air ou de l'oxygène.

**11.** Procédé selon la revendication précédente, caractérisé en ce que l'on effectue la réaction avec un mélange gazeux comprenant 0,1 à 99,9 % en mole d'oxygène, et plus particulièrement comprenant entre 1 et 90 % ou entre 97 et 99,9 % en mole d'oxygène.

**12.** Procédé selon l'une quelconque des revendications précédentes, caractérisé en ce que l'on utilise un mélange gazeux présentant un rapport molaire alcane/oxygène inférieur à 20 et plus particulièrement compris entre 0,01 et 0,2 ou entre 0,6 et 15.

**13.** Procédé selon l'une quelconque des revendications précédentes, caractérisé en ce que l'on utilise un mélange gazeux comprenant de l'eau.

**14.** Procédé selon la revendication précédente, caractérisé en ce que l'on utilise un mélange gazeux comprenant 0 à 70 % en mole d'eau, et de préférence comprenant 0 à 20 % en moles d'eau.

**15.** Procédé selon l'une quelconque des revendications précédentes, caractérisé en ce que l'on utilise un mélange gazeux comprenant un gaz diluant choisi parmi les gaz rares ou l'azote.

**16.** Procédé selon la revendication précédente, caractérisé en ce que l'on utilise un mélange gazeux comprenant 0 à 70 % en mole de gaz diluant et de préférence comprenant 0 à 20 % en moles dudit gaz.

**17.** Procédé selon l'une quelconque des revendications précédentes, caractérisé en ce que l'on fait réagir l'éthane pour obtenir l'acide acétique.


**Claims**

**1.** Process for the preparation of a saturated carboxylic acid by a gas phase reaction of the corresponding alkane with a source of oxygen, characterized in that the reaction is performed in the presence of a catalyst in which the active phase is based on vanadium, titanium, molybdenum, phosphorus and oxygen.

**2.** Process according to the preceding claim, characterized in that the reaction is performed in the presence of a catalyst in which the active phase corresponds to the following formula: $P_aMO_bV_cTi_dO_x$; in which the stoichiometric coefficients satisfy the following relationships:

$$b + c + d = 1.0;$$

$$0.9 < (a/c) < 3;$$

$$0.5 < d < 0.99;$$

and

$$0.01 < (c/b+c) < 0.92.$$

**3.** Process according to the preceding claim, characterized in that the stoichiometric coefficients satisfy the following equations:

$$b + c + d = 1.0;$$

$$1.0 < (a/c) < 2.2;$$

$$0.7 < d < 0.98;$$

and

$$0.08 < (c/b+c) < 0.6.$$

4. Process according to any one of the preceding claims, characterized in that a catalyst in bulk or diluted form is employed.

5. Process according to the preceding claim, characterized in that a coated catalyst is employed including 0.1 to 30 % by weight of active phase relative to the total weight of finished catalyst, and preferably from 2 to 20 % by weight.

6. Process according to claim 4, characterized in that a catalyst is employed including a support dispersed in the active phase, including 1 to 90 % of active phase relative to the total weight of the finished catalyst.

7. Process according to any one of the preceding claims, characterized in that a catalyst is employed in which the active phase furthermore includes at least one dopant chosen from the following elements:
K, Rb, Cs, Ca, Mg, Zr, Hf, Nb, Ta, Cr, W, Mn, Re, Fe, Ru, Os, Co, Rh, Ir, Ni, Pd, Cu, Ag, Zn, Cd, Tl, Si, Ge, Sn, As, Sb, Bi, Ga and the rare earths.

8. Process according to the preceding claim, characterized in that the dopant content is such that the ratio of the dopant to vanadium is between 0.005 and 0.10, and preferably between 0.009 and 0.06.

9. Process according to any one of the preceding claims, characterized in that the reaction is performed with a gaseous mixture including 0.1 to 99.9 mol% of alkane, and more particularly the said mixture includes between 0.1 and 3 mol% or between 10 and 99 mol% of alkane.

10. Process according to any one of the preceding claims, characterized in that air or oxygen is employed as a source of oxygen.

11. Process according to the preceding claim, characterized in that the reaction is performed with a gaseous mixture including 0.1 to 99.9 mol% of oxygen, and more particularly including between 1 and 90 mol% or between 97 and 99.9 mol% of oxygen.

12. Process according to any one of the preceding claims, characterized in that a gaseous mixture is employed exhibiting an alkane/oxygen molar ratio less than 20 and more particularly between 0.01 and 0.2 or between 0.6 and 15.

13. Process according to any one of the preceding claims, characterized in that a gaseous mixture including water is employed.

14. Process according to the preceding claim, characterized in that a gaseous mixture is employed including 0 to 70 mol% of water and preferably including 0 to 20 mol% of water.

15. Process according to any one of the preceding claims, characterized in that a gaseous mixture is employed including a diluent gas chosen from the rare gases or nitrogen.

16. Process according to the preceding claim, characterized in that a gaseous mixture is employed including 0 to 70 mol% of diluent gas and preferably including 0 to 20 mol% of the said gas.

17. Process according to any one of the preceding claims, characterized in that ethane is reacted to obtain acetic acid.

**Patentansprüche**

1. Verfahren zur Herstellung einer gesättigten Carbonsäure durch Umsetzung des entsprechenden Alkans mit einer Sauerstoffquelle in der Gasphase, dadurch gekennzeichnet, daß man die Umsetzung in Gegenwart eines Katalysators ausführt, dessen aktive Phase auf Vanadium, Titan, Molybdän, Phosphor und Sauerstoff basiert.

2. Verfahren nach dem vorangegangenen Anspruch, dadurch gekennzeichnet, daß man die Umsetzung in Gegenwart eines Katalysators bewirkt, dessen aktive Phase der folgenden Formel entspricht: $P_aMo_bV_cTi_dO_x$, in welcher die stöchiometrischen Koeffizienten die folgenden Beziehungen erfüllen:

$$b + c + d = 1,0;$$

$$0,9 < (a/c) < 3;$$

$$0,5 < d < 0,99$$

und

$$0,01 < (c/b+c) < 0,92.$$

3. Verfahren nach dem vorangegangenen Anspruch, dadurch gekennzeichnet, daß die stöchiometrischen Koeffizienten die folgenden Gleichungen erfüllen:

$$b + c + d = 1,0;$$

$$1,0 < (a/c) < 2,2;$$

$$0,7 < d < 0,98$$

und

$$0,08 < (c/b+c) < 0,6.$$

4. Verfahren nach einem der vorangegangenen Ansprüche, dadurch gekennzeichnet, daß man einen Katalysator in massiver oder in verdünnter Form einsetzt.

5. Verfahren nach dem vorangegangenen Anspruch, dadurch gekennzeichnet, daß man einen überzogenen Katalysator einsetzt, der 0,1 bis 30 Gew.-% aktive Phase bezogen auf das Gesamtgewicht des fertigen Katalysators und vorzugsweise 2 bis 20 Gew.-% umfaßt.

6. Verfahren nach Anspruch 4, dadurch gekennzeichnet, daß man einen Katalysator verwendet, der einen in der aktiven Phase dispergierten Träger umfaßt und 1 bis 90 % aktive Phase bezogen auf das Gesamtgewicht des fertigen Katalysators umfaßt.

7. Verfahren nach einem der vorangegangenen Ansprüche, dadurch gekennzeichnet, daß man einen Katalysator verwendet, dessen aktive Phase darüber hinaus mindestens ein Dotierungsmittel umfaßt, ausgewählt aus den folgenden Elementen: K, Rb, Cs, Ca, Mg, Zr, Hf, Nb, Ta, Cr, W, Mn, Re, Fe, Ru, Os, Co, Rh, Ir, Ni, Pd, Cu, Ag, Zn, Cd, Tl, Si, Ge, Sn, As, Sb, Bi, Ga und den Seltenerdelementen.

8. Verfahren nach dem vorangegangenen Anspruch, dadurch gekennzeichnet, daß der Gehalt an Dotierungsmittel so ist, daß das Verhältnis des Dotierungsmittels zu Vanadium zwischen 0,005 und 0,10 und vorzugsweise zwischen 0,009 und 0,06 beträgt.

**9.** Verfahren nach einem der vorangegangenen Ansprüche, dadurch gekennzeichnet, daß man die Umsetzung mit einer gasförmigen Mischung, umfassend 0,1 bis 99,9 mol-% Alkan, ausführt und die Mischung insbesondere 0,1 bis 3 mol-% oder 10 bis 99 mol-% Alkan umfaßt.

**10.** Verfahren nach einem der vorangegangenen Ansprüche, dadurch gekennzeichnet, daß man als Sauerstoffquelle Luft oder Sauerstoff verwendet.

**11.** Verfahren nach dem vorangegangenen Anspruch, dadurch gekennzeichnet, daß man die Umsetzung mit einer gasförmigen Mischung, umfassend 0,1 bis 99,9 mol-% Sauerstoff und insbesondere umfassend 1 bis 90 mol-% oder 97 bis 99,9 mol-% Sauerstoff, bewirkt.

**12.** Verfahren nach einem der vorangegangenen Ansprüche, dadurch gekennzeichnet, daß man eine gasförmige Mischung verwendet, die ein Molverhältnis Alkan/Sauerstoff unter 20 und insbesondere zwischen 0,01 und 0,2 oder zwischen 0,6 und 15 aufweist.

**13.** Verfahren nach einem der vorangegangenen Ansprüche, dadurch gekennzeichnet, daß man eine gasförmige Mischung, welche Wasser umfaßt, einsetzt.

**14.** Verfahren nach dem vorangegangenen Anspruch, dadurch gekennzeichnet, daß man eine gasförmige Mischung, welche 0 bis 70 mol-% Wasser und vorzugsweise 0 bis 20 mol-% Wasser umfaßt, einsetzt.

**15.** Verfahren nach einem der vorangegangenen Ansprüche, dadurch gekennzeichnet, daß man eine gasförmige Mischung, welche ein Verdünnungsgas, ausgewählt aus den Edelgasen oder Stickstoff, umfaßt, einsetzt.

**16.** Verfahren nach dem vorangegangenen Anspruch, dadurch gekennzeichnet, daß man eine gasförmige Mischung, welche 0 bis 70 mol-% Verdünnungsgas und vorzugsweise 0 bis 20 mol-% dieses Gases umfaßt, einsetzt.

**17.** Verfahren nach einem der vorangegangenen Ansprüche, dadurch gekennzeichnet, daß man Ethan reagieren läßt, um Essigsäure zu erhalten.